# EUROPÄISCHE PATENTSCHRIFT

(11) **EP 3 798 657 B1**
(45) Veröffentlichungstag und Bekanntmachung des Hinweises auf die Patenterteilung: **10.12.2025**
(21) Anmeldenummer: 19199992.9
(22) Anmeldetag: 27.09.2019
(51) Int. Cl.: G01R 33/483, G01R 33/54, G01R 33/565

(54) **ERFASSEN VON DATEN EINES UNTERSUCHUNGSOBJEKTES MITTELS MAGNETRESONANZ MIT VERBESSERTER ZEITEINTEILUNG**
DETECTION OF DATA OF AN EXAMINATION OBJECT USING MAGNETIC RESONANCE WITH IMPROVED TIME SCHEDULING
DÉTECTION DES DONNÉES D'UN OBJET D'ÉTUDE PAR RÉSONANCE MAGNÉTIQUE À INTÉGRATION TEMPORELLE AMÉLIORÉE

(43) Veröffentlichungstag der Anmeldung: 31.03.2021
(73) Patentinhaber: Siemens Healthineers AG, 91301 Forchheim (DE)
(72) Erfinder: Körzdörfer, Gregor, 91058 Erlangen (DE); Nittka, Mathias, 91083 Baiersdorf (DE); Speier, Peter, 91056 Erlangen (DE)
(74) Vertreter: Siemens Healthineers Patent Attorneys

(56) Entgegenhaltungen:
- US-A1- 2010 289 493
- US-A1- 2016 116 557
- US-A1- 2016 178 719
- KAY NEHRKE ET AL: "DREAM-a novel approach for robust, ultrafast, multislice B1 mapping", MAGNETIC RESONANCE IN MEDICINE, 17 January 2012 (2012-01-17), pages n/a - n/a, XP055027975, ISSN: 0740-3194, DOI: 10.1002/mrm.24158

## Beschreibung

Die Erfindung betrifft ein Verfahren zum Erfassen von Daten eines Untersuchungsobjektes mittels Magnetresonanz mit verbesserter Zeiteinteilung.

Die Magnetresonanz-Technik (im Folgenden steht die Abkürzung MR für Magnetresonanz) ist eine bekannte Technik, mit der Bilder vom Inneren eines Untersuchungsobjektes erzeugt werden können. Vereinfacht ausgedrückt wird hierzu das Untersuchungsobjekt in einem Magnetresonanzgerät in einem vergleichsweise starken statischen, homogenen Grundmagnetfeld, auch B₀-Feld genannt, mit Feldstärken von 0,2 Tesla bis 7 Tesla und mehr positioniert, so dass sich dessen Kernspins entlang des Grundmagnetfeldes orientieren. Zum Auslösen von Kernspinresonanzen werden hochfrequente Anregungspulse (RF-Pulse) in das Untersuchungsobjekt eingestrahlt, die ausgelösten Kernspinresonanzen als sogenannte k-Raumdaten gemessen und auf deren Basis MR-Bilder rekonstruiert oder Spektroskopiedaten ermittelt. Zur Ortskodierung der Messdaten werden dem Grundmagnetfeld schnell geschaltete magnetische Gradientenfelder überlagert. Die aufgezeichneten Messdaten, auch kurz nur als Daten bezeichnet, werden digitalisiert und als komplexe Zahlenwerte in einer k-Raum-Matrix abgelegt. Aus der mit Werten belegten k-Raum-Matrix ist z.B. mittels einer mehrdimensionalen Fourier-Transformation ein zugehöriges MR-Bild rekonstruierbar.

Mittels einer schichtselektiven Anregung durch HF-Anregungspulse mit einer entsprechenden Bandbreite und gleichzeitigem Schalten von Gradientenfeldern in Schichtselektionsrichtung, sogenannten Schichtselektionsgradienten, können gezielt nur Spins einer gewünschten räumlichen Schicht in dem Untersuchungsobjekt angeregt werden.

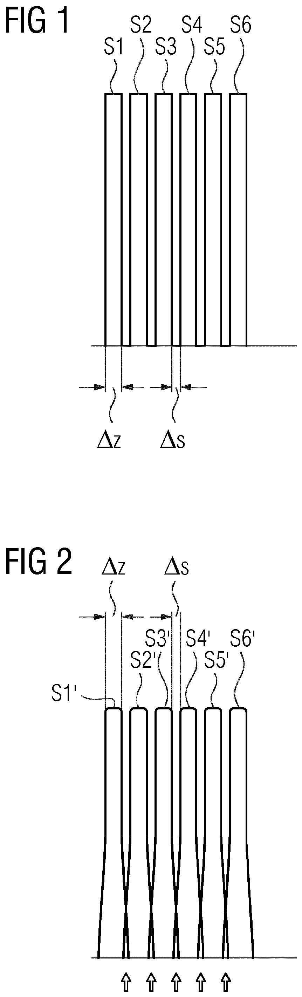

In Figur 1 sind schematisch ideale Schichtanregungsprofile einer derartigen schichtselektiven Anregung dargestellt. Die Darstellung zeigt beispielhaft sechs Schichten S1, S2, S3, S4, S5 und S6, die jeweils eine Ausdehnung in Schichtrichtung (die Schichtdicke) der Größe Δz aufweisen, die beispielsweise in einem Bereich von einem Millimeter bis zu einem Zentimeter oder ggf. auch mehr liegen kann. In dem gezeigten Beispiel haben die Schichten S1, S2, S3, S4, S5 und S6 jeweils in Schichtrichtung einen Abstand Δs voneinander. Je nach Anwendung kann der Abstand der verwendeten Schichten größer (z.B. größer oder gleich Δz) oder kleiner, auch gleich Null, oder negativ (sodass die Schichten überlappen) gewählt werden.

In der Realität ist es jedoch nicht möglich, ideale Schichtanregungsprofile mit idealer Rechteckfunktion (wie z.B. in Figur 1 dargestellt) anzuregen. Da ein realer HF-Anregungspuls immer eine endliche Bandbreite aufweist, wird ein reales Schichtanregungsprofil nie exakt rechtwinklig aussehen. In Figur 2 sind schematisch reale Schichtanregungsprofile S1', S2', S3', S4', S5' und S6' dargestellt, die analog zu Figur 1 eine Schichtdicke Δz und einen Abstand Δs voneinander aufweisen sollen. Die realen Schichtanregungsprofile S1', S2', S3', S4', S5' und S6' laufen im unteren dargestellten Bereich auseinander, sodass es zu Überschneidungen kommt (durch dicke Pfeile markiert).

Dies bedeutet, dass bei jeder Anregung einer Schicht aufgrund der endlichen Bandbreite des HF-Anregungspulses auch außerhalb der Schicht eine Anregung der Spins, und damit eine Magnetisierung der Spins in der Schicht räumlich benachbarten Bereichen, stattfindet. Man spricht hierbei von dem Problem des Schichtübersprechens (engl. "slice cross-talk") bei schichtselektiven MR-Messungen, insbesondere bei MR-Bildgebung.

Bei MR-Messungen mehrerer Schichten mit geringem Abstand Δs kann ein derartiges Übersprechen dazu führen, dass das Signal einer Schicht durch eine vorangehende Anregung einer benachbarten Schicht beeinflusst wird (dies wird auch Vorsättigungseffekt genannt), was zu Signalverlusten bzw. Kontraständerung führen kann.

Das Problem kann prinzipiell durch einen Anwender durch Wahl geeigneter Aufnahmeparameter (z.B. hinreichende Schichtabstände Δs, oder zeitliche Abstände zwischen den Schichtanregungen) adressiert werden. Allerdings sind dem Anwender die dafür notwendigen Kenntnisse (z.B. die Schichtanregungsprofile der jeweils verwendeten HF-Anregungspulse) in aller Regel nicht zugänglich, bzw. die physikalischen Zusammenhänge gar nicht bekannt. Daher ist es unwahrscheinlich, dass ein Anwender in der Lage ist, diesem Problem selbst abzuhelfen.

Die US 2016/0178719 A1 offenbart ein Verfahren zur Reduktion eines Schichtübersprechens bei Mehrschicht-MR-Messungen.

Es ist auch möglich, möglicherweise ungünstige Messparameter durch Einschränkungen in der Mess-Software zu verhindern. Beispielsweise kann ein Mindestabstand für den Schichtabstand Δs, z.B. 30% der Schichtdicke Δz, erzwungen werden, der ein Übersprechen verhindern oder zumindest verringern soll. Dies ist jedoch oftmals entgegen dem ausgesprochenen Willen eines Anwenders, der möglicherweise keine derartigen Lücken zwischen den gemessenen Schichten wünscht.

Der Erfindung liegt die Aufgabe zugrunde, schichtselektive MR-Messungen zu ermöglichen, die die oben genannten Nachteile vermeiden.

Die Aufgabe wird gelöst durch ein Verfahren zum Erfassen von Daten eines Untersuchungsobjektes mittels Magnetresonanz gemäß Anspruch 1, eine Magnetresonanzanlage gemäß Anspruch 13, ein Computerprogramm gemäß Anspruch 14, sowie einen elektronisch lesbaren Datenträger gemäß Anspruch 15.

Ein erfindungsgemäßes Verfahren zum Erfassen von Daten eines Untersuchungsobjektes mittels Magnetresonanz umfasst die Schritte:
- Laden eines Messprotokolls zur Erfassung von Daten eines Untersuchungsbereichs des Untersuchungsobjektes in mindestens zwei Schichten mittels einer Pulssequenz,
- Bestimmen eines zeitlichen Abstandes zwischen im Rahmen der Pulssequenz durchgeführten Anregungen benachbarter Schichten,
- Bestimmen eines zeitlichen Mindestabstandes zwischen im Rahmen der Pulssequenz durchgeführten Anregungen benachbarter Schichten auf Basis von Pulssequenzparametern, Gewebeparametern des Untersuchungsbereichs des Untersuchungsobjektes von welchem Daten mit der Pulssequenz erfasst werden sollen und wählbaren Qualitätsparametern,
- Ermitteln von bestimmten zeitlichen Abständen zwischen im Rahmen der Pulssequenz durchgeführten Anregungen benachbarter Schichten, die den zugehörigen bestimmten zeitlichen Mindestabstand unterschreiten als anzupassende zeitliche Abstände,
- Anpassen der ermittelten anzupassenden zeitlichen Abstände zwischen im Rahmen der Pulssequenz durchgeführten Anregungen benachbarter Schichten, derart, dass keiner der angepassten zeitlichen Abstände zwischen im Rahmen der angepassten Pulssequenz durchgeführten Anregungen benachbarter Schichten den bestimmten zeitlichen Mindestabstand unterschreitet,
- Erfassen von Daten des Untersuchungsobjektes unter Verwendung einer angepassten Pulssequenz, die die angepassten zeitlichen Abstände einhält.

Der Erfindung liegt unter anderem die Erkenntnis zugrunde, dass im Allgemeinen nicht alle Schichten gleichermaßen von Übersprecheffekten betroffen sind, z.B. Randschichten. Dies kann zu besonders störenden Signalsprüngen von Schicht zu Schicht führen. Ist von benachbarten Schichten die Rede, so sind, wenn nichts anderes angegeben ist, räumlich benachbarte Schichten gemeint, die derart nah benachbart sind, dass eine Anregung einer Magnetisierung in einer Schicht auch zu einer Magnetisierung der benachbarten Schicht führt.

Noch gravierender kann sich das Problem bei quantitativer Bildgebung auswirken, wenn Übersprecheffekte die quantitativen Parameterkarten verfälschen, insbesondere weil das Auftreten des Problems für den Anwender nicht erkenntlich ist, z.B. wenn er die Sequenzparameter ändert (z.B. Schichtzahl oder Schichtabstand).

Durch die erfindungsgemäße, insbesondere automatisch mögliche, Bestimmung eines zeitlichen Mindestabstandes zwischen im Rahmen der Pulssequenz durchgeführten Anregungen benachbarter Schichten und eine gezielte, insbesondere automatische, Anpassung nur von anzupassenden zeitlichen Abständen zwischen im Rahmen der Pulssequenz durchgeführten Anregungen benachbarter Schichten, kann einerseits eine Verfälschung von Messergebnissen geschickt vermieden werden, wobei andererseits die Messzeit des gewählten Messprotokolls nicht unnötig erhöht wird, und der Anwender bei der Wahl der anzuregenden Schichten nicht ungewollt eingeschränkt wird.

Eine erfindungsgemäße Magnetresonanzanlage umfasst eine Magneteinheit, eine Gradienteneinheit, eine Hochfrequenzeinheit und eine zur Durchführung eines erfindungsgemäßen Verfahrens ausgebildete Steuereinrichtung mit einer Abstandsbestimmungseinheit.

Ein erfindungsgemäßes Computerprogramm implementiert ein erfindungsgemäßes Verfahren auf einer Steuereinrichtung einer Magnetresonanzanlage, wenn es auf der Steuereinrichtung ausgeführt wird.

Das Computerprogramm kann hierbei auch in Form eines Computerprogrammprodukts vorliegen, welches direkt in einen Speicher einer Steuereinrichtung ladbar ist, mit Programmcode-Mitteln, um ein erfindungsgemäßes Verfahren auszuführen, wenn das Computerprogrammprodukt in der Steuereinrichtung einer Magnetresonanzanlage, ausgeführt wird.

Ein erfindungsgemäßer elektronisch lesbarer Datenträger umfasst darauf gespeicherte elektronisch lesbare Steuerinformationen, welche zumindest ein erfindungsgemäßes Computerprogramm umfassen und derart ausgestaltet sind, dass sie bei Ausführung der Steuerinformationen in einer Steuereinrichtung einer Magnetresonanzanlage ein erfindungsgemäßes Verfahren durchführen.

Die in Bezug auf das Verfahren angegebenen Vorteile und Ausführungen gelten analog auch für die Magnetresonanzanlage, das Computerprogramm und den elektronisch lesbaren Datenträger.

Weitere Vorteile und Einzelheiten der vorliegenden Erfindung ergeben sich aus den im Folgenden beschriebenen Ausführungsbeispielen sowie anhand der Zeichnungen. Die aufgeführten Beispiele stellen keine Beschränkung der Erfindung dar. Es zeigen:
- Fig. 1: eine schematische Darstellung idealer Schichtanregungsprofile,
- Fig. 2: eine schematische Darstellung realer Schichtanregungsprofile,
- Fig. 3: und 4 grob schematische Darstellungen von Ausschnitten vereinfachter Pulssequenzen,
- Fig. 5: eine schematische Darstellung eines Ablaufdiagramms eines erfindungsgemäßen Verfahrens,
- Fig. 6: eine schematisch dargestellte erfindungsgemäße Magnetresonanzanlage.

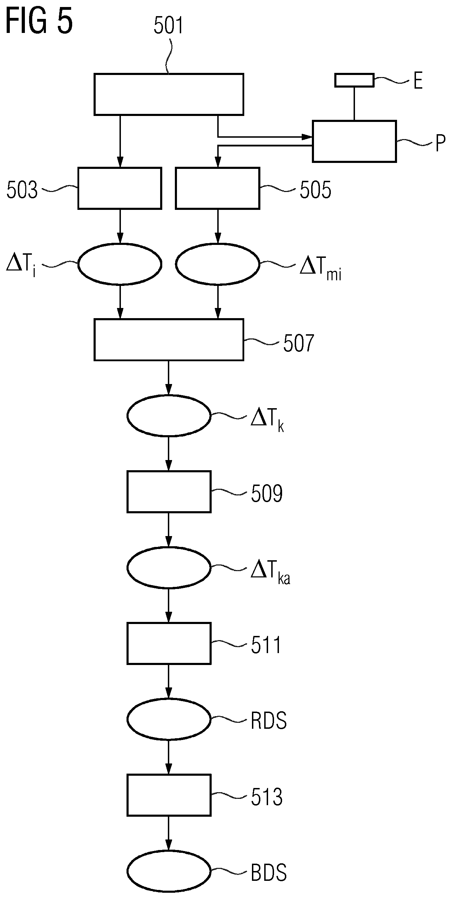

Figur 5 ist ein schematisches Ablaufdiagramm eines erfindungsgemäßen Verfahrens zum Erfassen von Daten eines Untersuchungsobjektes mittels Magnetresonanz.

Dabei wird ein Messprotokoll zur Erfassung von Daten eines Untersuchungsbereichs des Untersuchungsobjektes in mindestens zwei Schichten mittels einer Pulssequenz geladen (Block 501). Hierbei wird somit insbesondere festgelegt, aus welchem Untersuchungsbereich des Untersuchungsobjektes, z.B. von welchem anatomischen Untersuchungsbereich (z.B. Kopf, Herz, Leber, Gelenk, Ganzkörper, usw.) eines Patienten als Untersuchungsobjekt, und in welchen Schichten Daten erfasst werden sollen, und welche Pulssequenz dabei eingesetzt werden soll. Es wird ein zeitlicher Abstand ΔTᵢ zwischen im Rahmen der Pulssequenz durchgeführten Anregungen benachbarter Schichten der mindestens zwei Schichten bestimmt (Block 503). Dies kann z.B. basierend auf Pulssequenzparametern des geladenen Messprotokolls erfolgen, insbesondere aus einer in dem geladenen Messprotokoll festgelegten zeitlichen Abfolge von Anregungen. Hierbei liegt der Index i zwischen Eins (der kleinsten möglichen Anzahl an derartigen zeitlichen Abständen ΔTᵢ) und der Anzahl der Schichten, aus welchen Daten erfasst werden sollen, minus Eins (der größten möglichen Anzahl an derartigen zeitlichen Abständen ΔTᵢ). Werden aus N Schichten Daten mittels des Verfahrens erfasst, läuft der Index i also in ganzzahligen Schritten von 1 bis N-1.

Durch die Wahl des Messprotokolls, und damit des Untersuchungsbereichs, der Schichten und der Pulssequenz können sämtliche zeitlichen Abstände ΔTᵢ zwischen im Rahmen der Pulssequenz durchgeführten Anregungen benachbarter Schichten der mindestens zwei Schichten direkt aus dem geplanten Messablauf des geladenen Messprotokolls heraus abgelesen und damit bestimmt werden.

Darüber hinaus wird ein zeitlicher Mindestabstand ΔTₘᵢ zwischen im Rahmen der Pulssequenz durchgeführten Anregungen benachbarter Schichten auf Basis von Parametern P, Pulssequenzparametern, Gewebeparametern des Untersuchungsbereichs des Untersuchungsobjektes, von welchem Daten mit der Pulssequenz erfasst werden sollen, und wählbaren Qualitätsparametern, bestimmt (Block 505). Ein zeitlicher Abstand ΔTᵢ, der den zeitlichen Abstand der Anregungen zweier benachbarter Schichten im Rahmen der Pulssequenz wiedergibt, ist hierbei einem zeitlichen Mindestabstand ΔTₘᵢ zugehörig, der den zeitlichen Mindestabstand der Anregungen derselben benachbarten Schichten wiedergibt.

Der zeitliche Mindestabstand ΔTₘᵢ wird hierbei derart bestimmt, dass Artefakte, insbesondere durch Schichtübersprechen erzeugte Übersprechartefakte, bei dem Erfassen der Daten vermieden oder zumindest reduziert werden. Auf Basis des bestimmten zeitlichen Mindestabstands ΔTₘᵢ lässt sich ermitteln, ob zwischen zwei oder mehr Schichten der mindestens zwei Schichten signifikante Übersprecheffekte zu erwarten sind.

Bei dem Bestimmen eines zeitlichen Mindestabstands ΔTₘᵢ zwischen im Rahmen der Pulssequenz durchgeführten Anregungen benachbarter Schichten kann ein maximaler Wert für eine longitudinale Relaxationszeit T1 eines in dem zu untersuchenden Untersuchungsbereich vorliegenden und bei dem Erfassen der Daten angeregten Gewebes als Gewebeparameter angenommen werden. Dadurch kann sichergestellt werden, dass jegliche durch eine Anregung einer Schicht erzeugte Magnetisierung vor einer Anregung einer benachbarten Schicht abgeklungen ist. Beispielsweise kann eine maximale longitudinale Relaxationszeit T1 konservativ abgeschätzt werden als die längste longitudinale Relaxationszeit T1 eines im Untersuchungsbereich möglicherweise vorkommenden Gewebes, das bei einer Anregung angeregt wird. Für in einem menschlichen oder tierischen Patienten vorkommende Gewebe kann so als Wert für eine maximale longitudinale Relaxationszeit beispielsweise ca. 3 Sekunden gewählt werden.

Es ist auch denkbar, eine maximale longitudinale Relaxationszeit z.B. auf Basis des geladenen Messprotokolls und/oder des Untersuchungsbereichs, aus welchem Daten erfasst werden sollen, und z.B. unter Verwendung bereits gespeicherter Werte für longitudinale Relaxationszeiten zu bestimmen. Hierbei kann insbesondere auf Basis des Untersuchungsbereichs, z.B. unter Verwendung einer vorab gespeicherten Zuordnung von Geweben zu Untersuchungsbereichen, in welchen die Gewebe vorkommen, bestimmt werden, welche Gewebe in dem Untersuchungsbereich vorliegen. Auf Basis des geladenen Messprotokolls kann zusätzlich oder alternativ bestimmt werden, welche Gewebe in dem Untersuchungsbereich angeregt werden. Eine einem auf eine dieser Arten bestimmten Gewebe zugeordnete longitudinale Relaxationszeit T1 kann beispielsweise ebenfalls vorab z.B. als typische longitudinale Relaxationszeit T1 des jeweiligen Gewebes, oder als maximale longitudinale Relaxationszeit T1 des jeweiligen Gewebes oder auch als ein Bereich möglicher longitudinaler Relaxationszeiten T1 des jeweiligen Gewebes gespeichert vorliegen, um in Abhängigkeit des Messprotokolls und/oder des Untersuchungsbereichs, z.B. durch Ermitteln der längsten longitudinalen Relaxationszeit T1 der bestimmten Gewebe, eine maximale longitudinale Relaxationszeit T1 zu bestimmen.

Eine derartige Bestimmung einer maximalen longitudinalen Relaxationszeit T1 kann somit insbesondere automatisch erfolgen, wobei auf Basis des geladenen Messprotokolls und/oder auf Basis des Untersuchungsbereichs relevante Gewebe bestimmt werden können, und unter Verwendung von bereits gespeicherten Zuordnungen von longitudinalen Relaxationszeiten T1 zu möglichen Geweben automatisch eine maximale longitudinale Relaxationszeit T1 festgelegt werden kann.

Es ist auch möglich, dass ein Anwender die maximale longitudinale Relaxationszeit T1 frei wählt und durch eine Eingabe E als Parameter P festlegt. Hierbei sollte der Anwender jedoch über ausreichend Kenntnisse verfügen, um eine sinnvolle Wahl treffen zu können.

Die Parameter für das Bestimmen eines zeitlichen Mindestabstandes ΔTₘᵢ zwischen im Rahmen der Pulssequenz durchgeführten Anregungen benachbarter Schichten kann zusätzlich oder alternativ als Pulssequenzparameter ein Schichtanregungsprofil der zur Anregung der Schichten verwendeten HF-Anregungspulse umfassen. Auf Basis der aus der von dem geladenen Messprotokoll umfassten Pulssequenz bestimmbaren Schichtanregungsprofilen der zur Anregung der Schichten verwendeten HF-Anregungspulse kann insbesondere ein idealer Schichtabstand bestimmt werden, der zwischen benachbarten Schichten vorliegen sollte, um insbesondere Übersprechartefakte zu verhindern oder auf ein gewünschtes Maß (siehe auch weiter unten mit Bezug auf den Qualitätsparameter) zu reduzieren. Weiterhin kann aus Schichtanregungsprofilen ein Magnetisierungswert ermittelt werden, der angibt, wie stark eine Magnetisierung in einer Schicht sein kann, wenn eine benachbarte Schicht angeregt wird.

Die Parameter für das Bestimmen eines zeitlichen Mindestabstandes zwischen im Rahmen der Pulssequenz durchgeführten Anregungen benachbarter Schichten können zusätzlich oder alternativ als Qualitätsparameter einen Wert für eine maximal zulässige Vorsättigung einer Magnetisierung einer der Schichten vor ihrer Anregung, z.B. als prozentualen Wert, umfassen. Beispielsweise kann als Qualitätsparameter eine maximale Vorsättigung der longitudinalen Magnetisierung von z.B. 1% bis 3% oder 10% bis 20% oder sogar mehr vorgegeben werden. Die Wahl einer maximalen Vorsättigung kann beispielsweise je nach Anwendung erfolgen. Dabei können z.B. für eine qualitative MR-Messung höhere Werte für die Vorsättigung tolerierbar sein als z.B. für quantitative MR-Messungen, wie MRF. Ist in einer Anwendung ein Kontrast eines Gewebetyps mit einer langen longitudinalen Relaxationszeit T1, z.B. ein Flüssigkeitskontrast (Flüssigkeiten haben in verhältnismäßig lange longitudinale Relaxationszeiten T1), von nur untergeordneter Bedeutung, kann die maximale Vorsättigung entsprechend größer gewählt werden. Sind jedoch kleine Kontrastunterschiede in einem Gewebetyp mit langer longitudinaler Relaxationszeit T1 entscheidend für die Anwendung, sollte die maximale Vorsättigung entsprechend klein, z.B. auf ca. 1% bis ggf. 3%, begrenzt sein.

Durch derartige Qualitätsparameter kann eine höhere Flexibilität erreicht werden, insbesondere kann das Erfassen der Daten, wenn für eine Anwendung nicht die höchste grundsätzlich erreichbare Qualität erforderlich ist, in kürzerer Zeit und dabei noch in einer akzeptablen Qualität erfolgen. Der Qualitätsparameter kann beispielsweise anwendungsspezifisch, z.B. in Abhängigkeit des geladenen Messprotokolls, (automatisch) vorgegeben werden. Es ist auch möglich, dass ein Anwender durch eine Eingabe E den Qualitätswert Weise seinen Bedürfnissen nach vorgibt.

Parameter für das Bestimmen eines zeitlichen Mindestabstandes zwischen im Rahmen der Pulssequenz durchgeführten Anregungen benachbarter Schichten können somit von einem Nutzer für eine Erfassung von Daten des Untersuchungsobjektes, z.B. über Eingaben E, gewählt werden.

Parameter für das Bestimmen eines zeitlichen Mindestabstandes zwischen im Rahmen der Pulssequenz durchgeführten Anregungen benachbarter Schichten können aber auch zusätzlich oder alternativ vorbestimmt, z.B. in einem Speicher abrufbar hinterlegt, sein. Die vorbestimmten Parameter können aus Werten aus der Literatur abgeschätzt sein.

Zeitliche Abstände ΔTᵢ zwischen im Rahmen der Pulssequenz durchgeführten Anregungen benachbarter Schichten, die ihren jeweils zugehörigen bestimmten zeitlichen Mindestabstand ΔTₘᵢ unterschreiten, werden als anzupassende zeitliche Abstände ΔTₖ bestimmt (Block 507). Durch die Bestimmung anzupassender zeitlicher Abstände ΔTₖ wird insbesondere sichergestellt, dass nur zeitliche Abstände ΔTᵢ angepasst werden, die auch einer Anpassung zur Vermeidung von Artefakten bedürfen.

Der Index k läuft von dem Wert Eins, für einen ersten anzupassenden zeitlichen Abstand, bis zu dem letzten bestimmten anzupassenden zeitlichen Abstand, maximal also ebenfalls bis N-1.

Ermittelte anzupassende zeitliche Abstände ΔTₖ zwischen im Rahmen der Pulssequenz durchgeführten Anregungen benachbarter Schichten, werden derart zu angepassten zeitlichen Abständen ΔTₖₐ angepasst, dass keiner der angepassten zeitlichen Abstände ΔTₖ zwischen im Rahmen der angepassten Pulssequenz durchgeführten Anregungen benachbarter Schichten den zugehörigen bestimmten zeitlichen Mindestabstand ΔTₘᵢ der jeweiligen benachbarten Schichten unterschreitet.

In einem einfachen Fall kann das Anpassen eines anzupassenden bestimmten zeitlichen Abstands ΔTₖ ein Verlängern des bestimmten zeitlichen Abstands ΔTₖ auf mindestens den zugehörigen bestimmten zeitlichen Mindestabstand ΔTₘᵢ umfassen, sodass z.B. gilt: ΔTₘᵢ ≤ ΔTₖ + T_{w} = ΔTᵢ + T_{w} = ΔTₖₐ, wobei ein als anzupassender zeitlicher Abstand ΔTₖ bestimmter zeitlicher Abstand ΔTᵢ um eine Wartezeit T_{w} verlängert wurde, sodass als angepasster zeitlicher Abstand ΔTₖₐ mindestens der zugehörige zeitliche Mindestabstand ΔTₘᵢ erreicht wird.

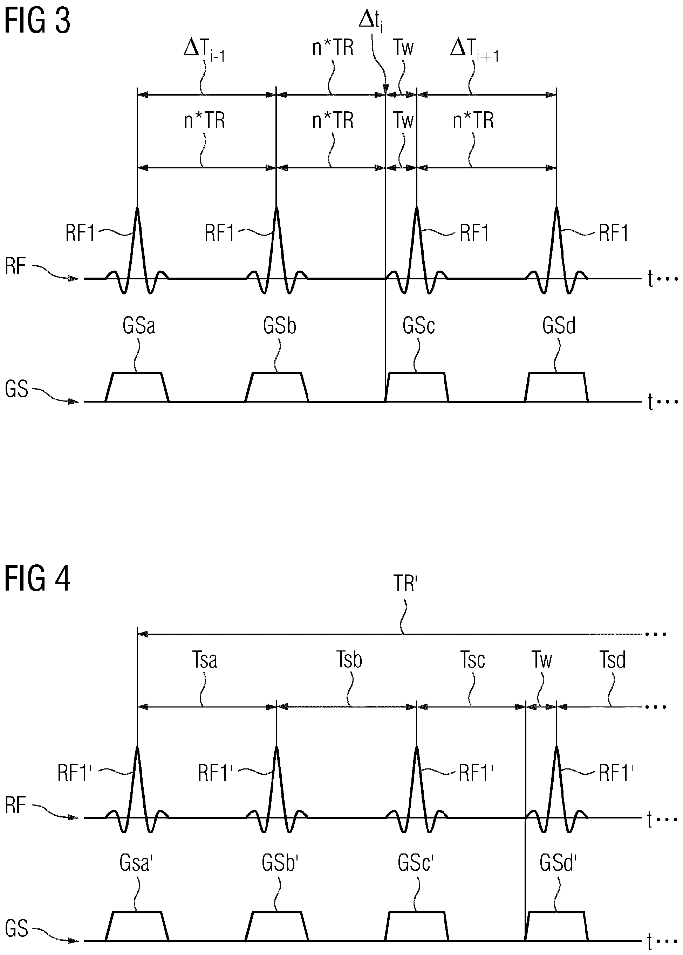

Die zu erfassenden Daten der mindestens zwei Schichten können beispielsweise nacheinander, jeweils für jede Schicht vollständig gemessen werden. Die Art der Erfassung der Daten wird durch das die Pulssequenz umfassende Messprotokoll festgelegt. Eine grob schematische Darstellung eines Ausschnitts einer derartigen Pulssequenz ist in Figur 3 wiedergegeben.

In Figur 3 ist schematisch in einer Zeile RF im Verlauf der Zeit t die zeitliche Reihenfolge von nacheinander eingestrahlten RF-Anregungspulsen RF1 dargestellt wie sie bei einer sequenziellen Mehrschicht("sequential multi-slice")-Messung ablaufen kann. Eine sequenzielle Mehrschicht-Messung nimmt somit die aufzunehmenden Schichten nacheinander auf. Während des Einstrahlens eines RF-Anregungspulses RF1 wird jeweils zeitlich abgestimmt ein Gradient GSa, GSb, GSc und GSd in Schichtselektionsrichtung GS geschaltet, um eine Anregung der Spins und damit eine Magnetisierung der Spins in einer dem jeweiligen RF-Anregungspuls RF1 und dem jeweiligen Gradienten GSa, GSb, GSc und GSd in Schichtselektionsrichtung GS entsprechenden Schicht zu erzeugen. In dem dargestellten Beispiel haben die gleichzeitig mit den Gradienten GSa, GSb, GSc und GSd in Schichtselektionsrichtung GS geschalteten RF-Anregungspulse RF1 jeweils eine andere (Mitten-)Frequenz, sodass jeweils eine andere von vier Schichten S1, S2, S3 und S4 angeregt wird, wobei beispielsweise der mit dem Gradienten GSa eingestrahlte RF-Anregungspuls RF1 eine Schicht S1, der mit dem Gradienten GSb eingestrahlte RF-Anregungspuls RF1 eine Schicht S3, der mit dem Gradienten GSc eingestrahlte RF-Anregungspuls RF1 eine Schicht S2 und der mit dem Gradienten GSd eingestrahlte RF-Anregungspuls RF1 eine Schicht S4 kodiert. In dem in Figur 3 dargestellten Beispiel haben alle Gradienten GSa, GSb, GSc und GSd in Schichtselektionsrichtung GS die gleiche Amplitude, was den üblichen Fall darstellt, dass für alle zu kodierenden Schichten RF-Anregungspulse RF' mit gleicher Anregungsbandbreite verwendet werden. Allgemein wäre es aber möglich, für jede Schicht mit unterschiedlichen Bandbreiten oder gar unterschiedlichen Anregungsprofilen der jeweiligen RF-Anregungspulse RF' zu arbeiten.

In der beispielhaft gezeigten Darstellung werden die Schichten in der Sortierung S1-S3-S2-S4 nacheinander angeregt, was eine übliche Sortierung darstellt, gemäß derer von den fortlaufend durchnummerierten Schichte zunächst von allen ungeradzahligen Schichten und darauffolgend von allen geradzahligen Schichten Daten erfasst werden. Gemäß eines geladenen Messprotokolls, das Daten mittels einer sequentiellen Mehrschicht-Messung aufnimmt, wären die jeweiligen Anregungen durch die Anregungspulse RF1 üblicherweise jeweils zeitlich gemäß der Wiederholzeit TR und der Anzahl n der pro Schicht aufzunehmenden Echosignalen voneinander beabstandet.

Beispielsweise bei einer Wiederholzeit TR von 10ms und n=1000 als Daten aufzunehmenden Echosignalen pro Schicht, ergibt sich eine Messzeit von 10s pro Schicht und damit zeitliche Abstände zwischen räumlich (unmittelbar) benachbarten Schichten für das in Figur 3 gezeigte Beispiel von ΔTᵢ₋₁ = 10s (zwischen den Schichten S1 und S3, da auf die letzte Anregung der Schicht S1 eine Anregung der Schicht S3 folgt, die keine unmittelbar benachbarte Schicht ist), und ΔTᵢ₊₁ = 10s (zwischen den Schichten S2 und S4, da auf die letzte Anregung der Schicht S2 eine Anregung der Schicht S4 folgt, die keine unmittelbar benachbarte Schicht ist), und ΔTᵢ = 0s (zwischen den Schichten S3 und S2, da auf die letzte Anregung der Schicht S3 eine Anregung der Schicht S2 folgt, die eine unmittelbar benachbarte Schicht ist).

Wurde für die Schichten S2 und S3 ein zeitlicher Mindestabstand ΔTₘᵢ, von beispielsweise 12s, bestimmt, kann der anzupassende zeitliche Abstand ΔTᵢ = ΔTₖ hier z.B. angepasst werden, indem eine Wartezeit T_{w}, die mindestens dem zugehörigen bestimmten Mindestabstand ΔTₘᵢ entspricht (T_{w} ≥ ΔTₘᵢ), bevorzugt genau dem zugehörigen bestimmten Mindestabstand ΔTₘᵢ entspricht (T_{w} = ΔTₘᵢ), zwischen der letzten Anregung der Schicht S3 und der ersten Anregung der Schicht S2 eingefügt werden. Dadurch wird der zeitliche Abstand ΔTᵢ um die Wartezeit T_{w} verlängert.

Z.B. auf diese Weise kann das Anpassen eines anzupassenden bestimmten zeitlichen Abstands ΔTₖ zwischen im Rahmen der Pulssequenz durchgeführten Anregungen benachbarter Schichten ein Einfügen einer Wartezeit T_{w} zwischen den Wiederholungen der Pulssequenz, in welchen je eine der benachbarten Schichten angeregt werden, deren Anregung einen anzupassenden zeitlichen Abstand ΔTₖ aufweist, umfassen.

Die Wartezeit T_{w} ist hierbei insbesondere derart gewählt, dass durch Einfügen der Wartezeit zu einem anzupassenden zeitlichen Abstand von Anregungen benachbarter Schichten der zeitliche Abstand der Anregungen der benachbarten Schichten derart angepasst wird, dass der angepasste zeitliche Abstand der Anregungen der benachbarten Schichten mindestens auf den zugehörigen Mindestabstand erhöht wird, bevorzugt auf genau den zugehörigen Mindestabstand erhöht wird.

Es ist auch denkbar, die zu erfassenden Daten der mindestens zwei Schichten zumindest teilweise innerhalb einer gemeinsamen eine Wiederholzeit langen Wiederholung der Pulssequenz zu erfassen, wie es beispielsweise im Rahmen einer verschachtelten segmentierten Mehrschicht("interleaved multi-slice")-Messung erfolgt. Eine grob schematische Darstellung eines Ausschnitts einer derartigen Pulssequenz ist in Figur 4 wiedergegeben. Hierbei können z.B. innerhalb einer Wiederholzeit TR' jeweils ein Echosignal einer Schicht durch nacheinander erfolgende schichtselektive Anregungen durch Einstrahlen von HF-Anregungspulsen RF1' unter gleichzeitigem Schalten von entsprechenden Gradienten GSa', GSb', GSc', GSd' in Schichtselektionsrichtung GS erzeugt und als Daten erfasst werden. Für die schichtselektive Anregung mittels der HF-Anregungspulse RF' und der Gradienten GSa', GSb', GSc', GSd' in Schichtselektionsrichtung GS gilt analog das mit Bezug auf Figur 3 ausgeführte. Sollen beispielsweise Daten aus sechs Schichten S1 bis S6 auf diese Weise erfasst werden, kann beispielsweise der mit dem Gradienten GSa' eingestrahlte RF-Anregungspuls RF1' die Schicht S1, der mit dem Gradienten GSb' eingestrahlte RF-Anregungspuls RF1' die Schicht S3, der mit dem Gradienten GSc' eingestrahlte RF-Anregungspuls RF1' die Schicht S5, der mit dem Gradienten GSd' eingestrahlte RF-Anregungspuls RF1' die Schicht S2 und weitere, nicht dargestellte Gradienten mit zugehörigen RF-Anregungspulsen die Schichten S4 und S6 kodieren.

Werden solche sechs (N=6) Schichten S1 bis S6 beispielsweise mit einer Wiederholzeit TR von 12s gemessen, ergeben sich für ein derartiges geladenes Messprotokoll üblicherweise zeitliche Anregungsabstände Tsa, Tsb, Tsc, Tsd zwischen zeitlich aufeinanderfolgend angeregten Schichten von jeweils TR/"Anzahl der Schichten", also z.B. im genannten Beispiel Tsa = Tsb = Tsc = Tsd = Ts =12s/6 = 2s, um die innerhalb einer Wiederholung zur Verfügung stehende Wiederholzeit TR gleichmäßig zu füllen.

Je nach Sortierung der Reihenfolge in welcher die Schichten S1 bis S6 angeregt werden sollen, ergeben sich die zeitlichen Abstände ΔTi von Anregungen unmittelbar benachbarter Schichten somit zunächst als Vielfache des oben genannten zeitlichen Anregungsabstands Ts (v*Ts, mit v = 1 ... N-1). In dem dargestellten Beispiel mit einer Sortierung der nacheinander anzuregenden Schichten gemäß dem oben beschriebenen Vorgehen gemäß S1-S3-S5-S2-S4-S6 ergäben sich somit für die zeitlichen Abstände ΔTᵢ der Anregungen unmittelbar benachbarter Schichten folgende Werte:
Tsa + Tsb + Tsc = 3*Ts, für den zeitlichen Abstand der Anregungen der Schichten S1 und S2,
Tsb + Tsc = 2*Ts, für den zeitlichen Abstand der Anregungen der Schichten S2 und S3,
Tsb + Tsc + Tsd = 3*Ts, für den zeitlichen Abstand der Anregungen der Schichten S3 und S4, und analog
2*Ts, für den zeitlichen Abstand der Anregungen der Schichten S4 und S5, und
3*Ts, für den zeitlichen Abstand der Anregungen der Schichten S5 und S6.

Wurde beispielsweise für zwei Schichten, z.B. die Schichten S2 und S3, ein zeitlicher Mindestabstand ΔTₘᵢ von 5s bestimmt, der in unserem obigen Beispiel mit Ts=2s, also länger als ΔTi(S2,S3) = 2*Ts = 4s ist, kann eine Wartezeit T_{w} z.B. mindestens der Länge ΔTₘᵢ - ΔTᵢ = 5s - 4s = 1s zwischen den Anregungen der betroffenen Schichten S2 und S3, hier also z.B. vor der Anregung der Schicht S2 durch den RF-Anregungspuls RF1', während welchem der Gradient GSd' geschaltet wird, eingefügt werden, um den anzupassenden zeitlichen Abstand ΔTₖ = ΔTᵢ(S2, S3) um die Wartezeit T_{w} auf mindestens den zugehörigen zeitlichen Mindestabstand ΔTₘᵢ zu verlängern (ΔTᵢ + T_{w} ≥ ΔTₘᵢ).

Somit kann das Anpassen eines anzupassenden bestimmten zeitlichen Abstands ΔTₖ von Anregungen benachbarter Schichten ein Einfügen einer Wartezeit T_{w} innerhalb einer Wiederholung der Pulssequenz zwischen den Anregungen der benachbarten Schichten umfassen.

Hierbei wurde vereinfachend angenommen, dass eine einzelne Teilmessung, also eine Erfassung der Daten eines Echosignals einer Schicht vernachlässigbar kurz ist (z.B. wenige Millisekunden). Falls die Erfassung der Daten eine nicht mehr vernachlässigbare Zeitdauer in Anspruch nimmt, müsste diese Zeitdauer mit auf die Wartezeit T_{w} aufgeschlagen werden.

Es kann also allgemein eine Wartezeit T_{w} derart nach einem anzupassenden zeitlichen Abstand ΔTₖ eingefügt werden, dass der so um die Wartezeit T_{w} verlängerte anzupassende zeitliche Abstand ΔTₖ+T_{w} den zugehörigen zeitlichen Mindestabstand ΔTₘᵢ nicht unterschreitet, bevorzugt dass der so um die Wartezeit verlängerte anzupassende zeitliche Abstand ΔTₖ+T_{w} dem zugehörigen zeitlichen Mindestabstand ΔTₘᵢ entspricht. Somit kann durch ein Einfügen der beschriebenen Wartezeiten ΔT_{w} eine hinreichende Relaxation der Magnetisierung im räumlichen Bereich einer folgenden Anregung einer Schicht gewährleistet und damit Übersprechartefakte vermieden werden. Da Wartezeiten T_{w} nur für anzupassende zeitliche Abstände ΔTₖ bestimmt und eingefügt werden, wird die insgesamte Messzeit nicht unnötig erhöht, sondern nur dort, wo es für ein Erreichen einer gewünschten Artefaktfreiheit erforderlich ist.

Durch ein Einfügen einer oben beschriebenen Wartezeit T_{w} ist kein Umsortieren der zeitlichen Reihenfolgen, in welcher die mindestens zwei Schichten angeregt werden, nötig, sodass mögliche andere durch das Umsortieren entstehen könnende Komplikationen vermieden werden. Das Einfügen einer Wartezeit T_{w} stellt nur einen minimalen Eingriff in den Ablauf der Pulssequenz dar. Es ist keine Iteration für eine derartige Anpassung der zeitlichen Abstände nötig, sondern eine Anpassung anzupassender zeitlicher Abstände durch Einfügen einer Wartezeit ist ohne großen Rechenaufwand möglich, sodass die angepassten zeitlichen Abstände schnell bestimmt werden können, um möglichst artefaktfrei Daten erfassen zu können.

Ein erfindungsgemäßes Verfahren ermöglicht eine, insbesondere automatische, Bestimmung von Wartezeiten T_{w}, die innerhalb einer von einem geladenen Messprotokoll umfassten Pulssequenz eingefügt werden können, um z.B. Störungen durch Schichtübersprechen zu vermeiden.

Zusätzlich oder alternativ ist es auch möglich, dass das Anpassen eines anzupassenden bestimmten zeitlichen Abstands ΔTₖ ein Umsortieren einer zeitlichen Reihenfolge, in welcher die mindestens zwei Schichten im Rahmen der Pulssequenz nacheinander angeregt werden, umfasst. Durch das Umsortieren wird ebenfalls ein zeitlicher Abstand der Anregungen zweier räumlich benachbarter Schichten verändert. Im Idealfall ist es möglich alle Schichten, aus welchen Daten erfasst werden sollen, derart anzuordnen, dass die zeitlichen Abstände der Anregungen räumlich benachbarter Schichten den bestimmten zeitlichen Mindestabstand einhalten. Allerdings kann das Umsortieren zu anderen Komplikationen, wie z.B. Wirbelstromeffekte, führen und das Auffinden einer solchen idealen Sortierung kann zeitaufwendig sein. Um sicherzustellen, dass nach einem Umsortieren der Anregungen der mindestens zwei Schichten nicht ein bisher nicht anzupassender zeitlicher Abstand ΔTᵢ zu einem anzupassenden zeitlichen Abstand ΔTₖ geworden ist, sollte nach einem Umsortieren erneut ein Durchlauf des Verfahrens (Block 501) begonnen werden. Somit kann iterativ eine ideale Sortierung gefunden werden.

Unter Verwendung einer angepassten Pulssequenz, die die angepassten zeitlichen Abstände einhält, werden gemäß dem geladenen Messprotokoll Daten RDS der mindestens zwei Schichten erfasst (Block 511).

Aus den erfassten Daten RDS können, z.B. mittels einer Fouriertransformation, Bilddaten BDS mindestens einer der mindestens zwei Schichten rekonstruiert werden (Block 513).

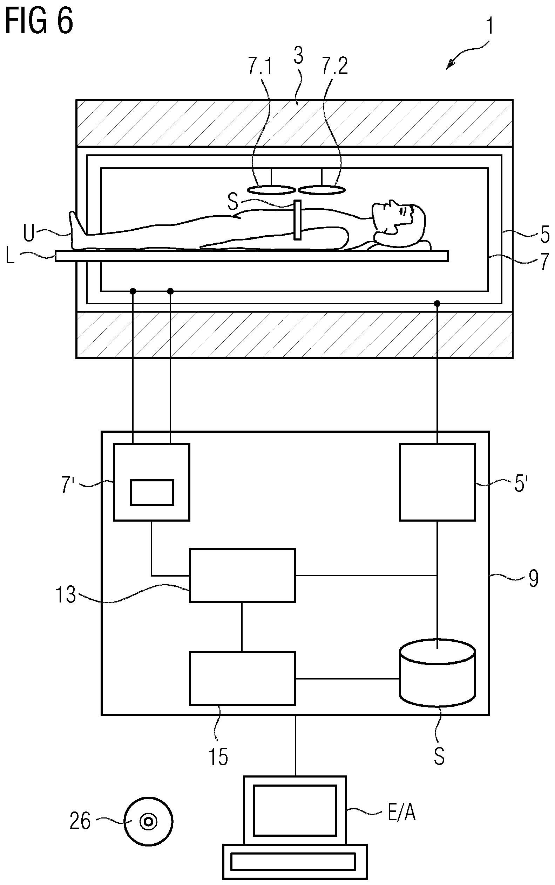

Figur 6 stellt schematisch eine erfindungsgemäße Magnetresonanzanlage 1 dar. Diese umfasst eine Magneteinheit 3 zur Erzeugung des Grundmagnetfeldes, eine Gradienteneinheit 5 zur Erzeugung der Gradientenfelder, eine Hochfrequenzeinheit 7 zur Einstrahlung und zum Empfang von Hochfrequenzsignalen und eine zur Durchführung eines erfindungsgemäßen Verfahrens ausgebildete Steuereinrichtung 9.

In der Figur 6 sind diese Teileinheiten der Magnetresonanzanlage 1 nur grob schematisch dargestellt. Insbesondere kann die Hochfrequenzeinheit 7 aus mehreren Untereinheiten, beispielsweise aus mehreren Spulen wie den schematisch gezeigten Spulen 7.1 und 7.2 oder mehr Spulen bestehen, die entweder nur zum Senden von Hochfrequenzsignalen oder nur zum Empfangen der ausgelösten Hochfrequenzsignale oder für beides ausgestaltet sein können.

Zur Untersuchung eines Untersuchungsobjektes U, beispielsweise eines Patienten oder auch eines Phantoms, kann dieses auf einer Liege L in die Magnetresonanzanlage 1 in deren Messvolumen eingebracht werden. Die Schicht S stellt ein exemplarisches Zielvolumen des Untersuchungsobjekts dar, aus dem Daten aufgenommen und damit erfasst werden sollen.

Die Steuereinrichtung 9 dient der Steuerung der Magnetresonanzanlage 1 und kann insbesondere die Gradienteneinheit 5 mittels einer Gradientensteuerung 5' und die Hochfrequenzeinheit 7 mittels einer Hochfrequenz-Sende-/Empfangs-Steuerung 7' steuern. Die Hochfrequenzeinheit 7 kann hierbei mehrere Kanäle umfassen, auf denen Signale gesendet oder empfangen werden können.

Die Hochfrequenzeinheit 7 ist zusammen mit ihrer Hochfrequenz-Sende-/Empfangs-Steuerung 7' für die Erzeugung und das Einstrahlen (Senden) eines Hochfrequenz-Wechselfeldes zur Manipulation der Spins in einem zu manipulierenden Bereich (beispielsweise in zu messenden Schichten S) des Untersuchungsobjekts U zuständig. Dabei wird die Mittenfrequenz des, auch als B1-Feld bezeichneten, Hochfrequenz-Wechselfeldes in aller Regel möglichst so eingestellt, dass sie nahe der Resonanzfrequenz der zu manipulierenden Spins liegt. Abweichungen von der Mittenfrequenz von der Resonanzfrequenz werden als Off-Resonanz bezeichnet. Zur Erzeugung des B1-Feldes werden in der Hochfrequenzeinheit 7 mittels der Hochfrequenz-Sende-/Empfangs-Steuerung 7' gesteuerte Ströme an den HF-Spulen angelegt.

Weiterhin umfasst die Steuereinrichtung 9 eine Abstandsbestimmungseinheit 15, mit welcher erfindungsgemäß zeitliche Abstände und/oder zeitliche Mindestabstände bestimmt werden können. Die Steuereinrichtung 9 ist insgesamt dazu ausgebildet, ein erfindungsgemäßes Verfahren durchzuführen.

Eine von der Steuereinrichtung 9 umfasste Recheneinheit 13 ist dazu ausgebildet alle für die nötigen Messungen und Bestimmungen nötigen Rechenoperationen auszuführen. Hierzu benötigte oder hierbei ermittelte Zwischenergebnisse und Ergebnisse können in einer Speichereinheit S der Steuereinrichtung 9 gespeichert werden. Die dargestellten Einheiten sind hierbei nicht unbedingt als physikalisch getrennte Einheiten zu verstehen, sondern stellen lediglich eine Untergliederung in Sinneinheiten dar, die aber auch z.B. in weniger oder auch in nur einer einzigen physikalischen Einheit realisiert sein können.

Über eine Ein-/Ausgabeeinrichtung E/A der Magnetresonanzanlage 1 können, z.B. durch einen Nutzer, Steuerbefehle an die Magnetresonanzanlage geleitet werden und/oder Ergebnisse der Steuereinrichtung 9 wie z.B. Bilddaten angezeigt werden.

Ein hierin beschriebenes Verfahren kann auch in Form eines Computerprogrammprodukts vorliegen, welches ein Programm umfasst und das beschriebene Verfahren auf einer Steuereinrichtung 9 implementiert, wenn es auf der Steuereinrichtung 9 ausgeführt wird. Ebenso kann ein elektronisch lesbarer Datenträger 26 mit darauf gespeicherten elektronisch lesbaren Steuerinformationen vorliegen, welche zumindest ein solches eben beschriebenes Computerprogrammprodukt umfassen und derart ausgestaltet sind, dass sie bei Ausführung der Steuerinformationen in einer Steuereinrichtung 9 einer Magnetresonanzanlage 1 das beschriebene Verfahren durchführen.

## Patentansprüche

1. Verfahren zum Erfassen von Daten eines Untersuchungsobjektes mittels Magnetresonanz (MR) umfassend die Schritte:
- Laden eines Messprotokolls zur Erfassung von Daten eines Untersuchungsbereichs des Untersuchungsobjektes in mindestens zwei Schichten mittels einer Pulssequenz,
- Bestimmen eines zeitlichen Abstandes zwischen im Rahmen der Pulssequenz durchgeführten Anregungen benachbarter Schichten,
- Bestimmen eines zeitlichen Mindestabstandes zwischen im Rahmen der Pulssequenz durchgeführten Anregungen benachbarter Schichten auf Basis von Pulssequenzparametern, Gewebeparametern des Untersuchungsbereichs des Untersuchungsobjektes von welchem Daten mit der Pulssequenz erfasst werden sollen und wählbaren Qualitätsparametern,
- Ermitteln von bestimmten zeitlichen Abständen zwischen im Rahmen der Pulssequenz durchgeführten Anregungen benachbarter Schichten, die den zugehörigen bestimmten zeitlichen Mindestabstand unterschreiten als anzupassende zeitliche Abstände,
- Anpassen der ermittelten anzupassenden zeitlichen Abstände zwischen im Rahmen der Pulssequenz durchgeführten Anregungen benachbarter Schichten, derart, dass keiner der angepassten zeitlichen Abstände zwischen im Rahmen der angepassten Pulssequenz durchgeführten Anregungen benachbarter Schichten den bestimmten zeitlichen Mindestabstand unterschreitet,
- Erfassen von Daten des Untersuchungsobjektes unter Verwendung einer angepassten Pulssequenz, die die angepassten zeitlichen Abstände einhält.

2. Verfahren nach Anspruch 1, wobei die zu erfassenden Daten der mindestens zwei Schichten nacheinander, jeweils für jede Schicht sequenziell gemessen werden.

3. Verfahren nach Anspruch 2, wobei das Anpassen eines anzupassenden bestimmten zeitlichen Abstands ein Einfügen einer Wartezeit zwischen zwei Wiederholungen der Pulssequenz umfasst.

4. Verfahren nach Anspruch 1, wobei die zu erfassenden Daten der mindestens zwei Schichten zumindest teilweise innerhalb einer gemeinsamen eine Wiederholzeit langen Wiederholung der Pulssequenz erfasst werden.

5. Verfahren nach Anspruch 4, wobei das Anpassen eines anzupassenden bestimmten zeitlichen Abstands ein Einfügen einer Wartezeit innerhalb einer Wiederholung der Pulssequenz umfasst.

6. Verfahren nach einem der Ansprüche 3 oder 5, wobei die Wartezeit derart nach einem anzupassenden zeitlichen Abstand eingefügt wird, dass der so um die Wartezeit verlängerte anzupassende zeitliche Abstand den zugehörigen zeitlichen Mindestabstand nicht unterschreitet, bevorzugt dass der so um die Wartezeit verlängerte anzupassende zeitliche Abstand dem zugehörigen zeitlichen Mindestabstand entspricht.

7. Verfahren nach einem der vorhergehenden Ansprüche, wobei das Anpassen eines anzupassenden bestimmten zeitlichen Abstands ein Umsortieren einer zeitlichen Reihenfolge, in welcher die mindestens zwei Schichten im Rahmen der Pulssequenz nacheinander angeregt werden, umfasst.

8. Verfahren nach einem der vorhergehenden Ansprüche, wobei bei dem Bestimmen eines zeitlichen Mindestabstandes zwischen im Rahmen der Pulssequenz durchgeführten Anregungen benachbarter Schichten ein maximaler Wert für eine longitudinale Relaxationszeit T1 eines in dem zu untersuchenden Untersuchungsbereich vorliegenden Gewebes als Gewebeparameter angenommen wird.

9. Verfahren nach einem der vorhergehenden Ansprüche, wobei die Parameter für das Bestimmen eines zeitlichen Mindestabstandes zwischen im Rahmen der Pulssequenz durchgeführten Anregungen benachbarter Schichten als Pulssequenzparameter ein Schichtanregungsprofil der zur Anregung der Schichten verwendeten HF-Anregungspulse umfasst.

10. Verfahren nach einem der vorhergehenden Ansprüche, wobei die Parameter für das Bestimmen eines zeitlichen Mindestabstandes zwischen im Rahmen der Pulssequenz durchgeführten Anregungen benachbarter Schichten als Qualitätsparameter einen Wert für eine maximal zulässige Vorsättigung einer Magnetisierung einer der Schichten vor ihrer Anregung umfasst.

11. Verfahren nach einem der vorhergehenden Ansprüche, wobei Parameter für das Bestimmen eines zeitlichen Mindestabstandes zwischen im Rahmen der Pulssequenz durchgeführten Anregungen benachbarter Schichten durch einen Nutzer für eine Erfassung von Daten des Untersuchungsobjektes gewählt werden.

12. Verfahren nach einem der vorhergehenden Ansprüche, wobei Parameter für das Bestimmen eines zeitlichen Mindestabstandes zwischen im Rahmen der Pulssequenz durchgeführten Anregungen benachbarter Schichten vorbestimmt sind.

13. Magnetresonanzanlage (1) umfassend, eine Magneteinheit (3), eine Gradienteneinheit (5), eine Hochfrequenzeinheit (7) und eine Steuereinrichtung (9) mit einer Hochfrequenz-Sende-/Empfangs-Steuerung (7') und einer Abstandsbestimmungseinheit (15), wobei die Steuereinrichtung (9) dazu ausgebildet ist, ein Verfahren nach einem der Ansprüche 1 bis 12 auf der Magnetresonanzanlage (1) auszuführen.

14. Computerprogramm, welches direkt in einen Speicher einer Steuereinrichtung (9) einer Magnetresonanzanlage (1) ladbar ist, mit Programm-Mitteln, um die Schritte des Verfahrens nach einem der Ansprüche 1 bis 12 auszuführen, wenn das Programm in der Steuereinrichtung (9) der Magnetresonanzanlage (1) ausgeführt wird.

15. Elektronisch lesbarer Datenträger mit darauf gespeicherten elektronisch lesbaren Steuerinformationen, welche zumindest ein Computerprogramm nach Anspruch 14 umfassen und derart ausgestaltet sind, dass sie bei Ausführung der Steuerinformationen in einer Steuereinrichtung (9) einer Magnetresonanzanlage (1) ein Verfahren nach einem der Ansprüche 1 bis 12 durchführen.

## Claims

1. Method for acquisition of data of an examination object by means of magnetic resonance (MR) comprising the steps:
- Loading a measurement protocol for acquisition of data of an examination region of the examination object in at least two slices by means of a pulse sequence,
- Determination of a time interval between excitations of neighbouring slices carried out within the framework of the pulse sequence,
- Determination of a minimum time interval between excitations of neighbouring slices carried out within the framework of the pulse sequence on the basis of pulse sequence parameters, tissue parameters of the examination region of the examination object from which data is to be acquired with the pulse sequence and selectable quality parameters,
- Establishing of specific time intervals between excitations of neighbouring slices carried out within the framework of the pulse sequence, which fall below the associated minimum time interval determined as time intervals to be adapted,
- Adaptation of the established time intervals to be adapted between excitations of neighbouring slices carried out within the framework of the pulse sequence in such a way that none of the adapted time intervals between excitations of neighbouring slices carried out within the framework of the adapted pulse sequence falls below the minimum time interval specified,
- Acquisition of data of the examination object using an adapted pulse sequence, which adheres to the adapted time intervals.

2. Method according to claim 1, wherein the data of at least two slices to be acquired is measured one after another, in each case sequentially for each slice.

3. Method according to claim 2, wherein the adaptation of a time interval determined that is to be adapted comprises an insertion of a wait time between two repetitions of the pulse sequence.

4. Method according to claim 1, wherein the data to be acquired of the at least two slices is acquired at least partly within a repetition of the pulse sequence with an overall length of one repetition time.

5. Method according to claim 4, wherein the adaptation of a time interval determined that is to be adapted comprises an insertion of a wait time within a repetition of the pulse sequence.

6. Method according to one of claims 3 or 5, wherein the wait time is inserted after a time interval to be adapted in such a way that the time interval to be adapted lengthened by the wait time in this way does not fall below the associated minimum time interval, preferably so that the time interval to be adapted lengthened by the wait time in this way corresponds to the associated minimum time interval.

7. Method according to one of the preceding claims, wherein the adaptation of a time interval determined that is to be adapted comprises a resorting of a chronological sequence in which the at least two slices are excited one after another within the framework of the pulse sequence.

8. Method according to one of the preceding claims, wherein, in the determination of a minimum time interval between excitations of neighbouring slices carried out within the framework of the pulse sequence, a maximum value for a longitudinal relaxation time T1 of tissue present in the examination region to be examined is assumed as the tissue parameter.

9. Method according to one of the preceding claims, wherein the parameters for the determination of a minimum time interval between excitations of neighbouring slices carried out within the framework of the pulse sequence comprise as a pulse sequence parameter a slice excitation profile of the RF excitation pulses used for excitation of the slices.

10. Method according to one of the preceding claims, wherein the parameters for the determination of a minimum time interval between excitations of neighbouring slices carried out within the framework of the pulse sequence comprise as a quality parameter a value for a maximum allowable presaturation of a magnetisation of one of the slices before its excitation.

11. Method according to one of the preceding claims, wherein the parameters for the determination of a minimum time interval between excitations of neighbouring slices carried out within the framework of the pulse sequence are chosen by a user for an acquisition of data of the examination object.

12. Method according to one of the preceding claims, wherein the parameters for the determination of a minimum time interval between excitations of neighbouring slices carried out within the framework of the pulse sequence are predefined.

13. Magnetic resonance apparatus (1) comprising a magnet unit (3), a gradient unit (5), a radio frequency unit (7) and a control device (9) with a radio-frequency transceiver control (7') and a distance determination unit (15), wherein the control device (9) is embodied to carry out a method according to one of claims 1 to 12 on the magnetic resonance apparatus (1).

14. Computer program, which is able to be loaded directly into a memory of a control device (9) of a magnetic resonance apparatus (1), with program means for carrying out the steps of the method according to one of claims 1 to 12 when the program is executed in the control device (9) of the magnetic resonance apparatus (1).

15. Electronically-readable data medium with electronically-readable control information stored thereon, which comprises at least one computer program according to claim 14 and is embodied in such a way that, when the control information is executed in a control device (9) of a magnetic resonance apparatus (1), it carries out a method according to one of claims 1 to 12.

## Revendications

1. Procédé de saisie de données d'un objet à examiner, au moyen d'une résonance magnétique (RM) comprenant les stades :
- charge d'un protocole de mesure pour la saisie de données d'une partie à examiner de l'objet à examiner en au moins deux couches au moyen d'une séquence d'impulsions,
- détermination d'une distance dans le temps entre des excitations, effectuées dans le cadre de la séquence d'impulsions, de couches voisines,
- détermination d'une distance minimum dans le temps entre des excitations, effectuées dans le cadre de la séquence d'impulsions, de couches voisines sur la base de paramètres de séquence d'impulsions, de paramètres de tissu de la partie à examiner de l'objet à examiner, dont des données doivent être saisies par la séquence d'impulsions et de paramètres de qualité pouvant être sélectionnés,
- détermination de distances dans le temps déterminées entre des excitations, effectuées dans le cadre de la séquence d'impulsions, de couches voisines, qui sont plus petites que la distance minimum dans le temps déterminée, qui leur appartient, comme distances dans le temps à adapter,
- adaptation des distances dans le temps à adapter déterminées entre des excitations, effectuées dans le cadre de la séquence d'impulsions, de couches voisines, de manière à ce qu'aucune des distances dans le temps adaptées entre des excitations, effectuées dans le cadre de la séquence d'impulsions adaptée, de couches voisines ne soit inférieure à la distance minium dans le temps déterminée,
- saisie de données de l'objet à examiner en utilisant une séquence d'impulsions adaptée, qui respecte les distances dans le temps adaptées.

2. Procédé suivant la revendication 1, dans lequel on mesure les données à saisir des au moins deux couches les unes après les autres, séquentiellement respectivement pour chaque couche.

3. Procédé suivant la revendication 2, dans lequel l'adaptation d'une distance dans le temps déterminée à adapter comprend une insertion d'un temps d'attente entre deux répétitions de la séquence d'impulsions.

4. Procédé suivant la revendication 1, dans lequel on saisit les données à saisir des au moins deux couches au moins en partie dans une répétition de temps de répétition de longueur commune de la séquence d'impulsions.

5. Procédé suivant la revendication 4, dans lequel l'adaptation d'une distance dans le temps déterminée à adapter comprend une insertion d'un temps d'attente dans une répétition de la séquence d'impulsions.

6. Procédé suivant l'une des revendications 3 ou 5, dans lequel on insère le temps d'attente après une distance dans le temps à adapter, de manière à ce que la distance dans le temps à adapter ainsi prolongée du temps d'attente ne soit pas inférieure à la distance minimum dans le temps lui appartenant, de préférence, de manière à ce que la distance dans le temps à adapter prolongée du temps d'attente corresponde à la distance minimum dans le temps, qui lui appartient.

7. Procédé suivant l'une des revendications précédentes, dans lequel l'adaptation de la distance dans le temps déterminée à adapter, comprend un tri d'une succession dans le temps, dans laquelle les au moins deux couches sont excitées l'une après l'autre dans le cadre de la séquence d'impulsions.

8. Procédé suivant l'une des revendications précédentes, dans lequel, lors de la détermination d'une distance minimum dans le temps entre des excitations, effectuées dans le cadre de la séquence d'impulsions, de couches voisines, on prend comme paramètre de tissu une valeur maximum d'un temps T1 de relaxation longitudinale d'un tissu présent dans la partie d'examen à examiner.

9. Procédé suivant l'une des revendications précédentes, dans lequel le paramètre pour la détermination d'une distance minimum dans le temps entre des excitations, effectuées dans le cadre de la séquence d'impulsions, de couches voisines, comprend comme paramètre de séquence d'impulsions, un profil d'excitation de couche des impulsions d'excitation HF utilisées pour l'excitation des couches.

10. Procédé suivant l'une des revendications précédentes, dans lequel le paramètre pour la détermination d'une distance minimum dans le temps entre des excitations, effectuées dans le cadre de la séquence d'impulsions, de couches voisines comprend comme paramètre de qualité une valeur d'une pré-saturation admissible au maximum d'une magnétisation de l'une des couches avant son excitation.

11. Procédé suivant l'une des revendications précédentes, dans lequel les paramètres pour la détermination d'une distance minimum dans le temps entre des excitations, effectuées dans le cadre de la séquence d'impulsions, de couches voisines, sont choisis par un utilisateur pour une saisie de données de l'objet à examiner.

12. Procédé suivant l'une des revendications précédentes, dans lequel des paramètres pour la détermination d'une distance minimum dans le temps entre des excitations, effectuées dans le cadre de la séquence d'impulsions, de couches voisines, sont déterminés à l'avance.

13. Installation (1) de résonance magnétique, comprenant une unité (3) magnétique, une unité (5) de gradient, une unité (7) de haute fréquence et un dispositif (9) de commande ayant un dispositif (7') d'émission/réception de haute fréquence et une unité (15) de détermination de distance, dans laquelle le dispositif (9) de commande est constitué pour exécuter un procédé suivant l'une des revendications 1 à 12 sur l'installation (1) de résonance magnétique.

14. Programme d'ordinateur, qui peut être chargé directement dans la mémoire d'un dispositif (9) de commande d'une installation (1) de résonance magnétique, comprenant des moyens de programme pour exécuter les stades du procédé suivant l'une des revendications 1 à 12, lorsque le programme est exécuté dans le dispositif (9) de commande de l'installation (1) de résonance magnétique.

15. Support de données déchiffrable électroniquement, sur lequel sont mises en mémoire des informations de commande pouvant être déchiffrées électroniquement et qui comprennent au moins un programme d'ordinateur suivant la revendication 14 et sont conformées de manière à ce qu'elles exécutent, lors de l'exécution des informations de commande d'un dispositif (9) de commande d'une installation (1) de résonance magnétique, un procédé suivant l'une des revendications 1 à 12.
